# FASCICULE DE BREVET EUROPEEN

(11) **EP 3 177 357 B1**
(45) Date de publication et mention de la délivrance du brevet: **02.08.2023**
(21) Numéro de dépôt: 15760212.9
(22) Date de dépôt: 04.08.2015
(51) Int. Cl.: A61M 25/06

(54) **DISPOSITIF POUR L'INTRODUCTION D'UNE CANULE À BOUT ARRONDI SOUS LA PEAU D'UN PATIENT ET KIT**
VORRICHTUNG ZUM EINFÜHREN EINER KANÜLE MIT STUMPFER SPITZE UNTER DIE HAUT EINES PATIENTEN UND KIT
DEVICE FOR INSERTING A BLUNT-TIP CANNULA UNDER THE SKIN OF A PATIENT AND KIT

(30) Priorité: 04.08.2014 FR 1457592
(43) Date de publication de la demande: 14.06.2017
(73) Titulaire: Soft Medical Aesthetics, 75017 Paris (FR)
(72) Inventeur: SEBBAN Sandrine, 92300 Levallois Perret (FR); CORBIN Jean Yves, 14480 Le Fresne Camilly (FR); VAUPRES Maxime, 14540 Grentheville (FR); D'ESTAIS Mathias, 14000 Caen (FR); JEANSON Isabelle, 75016 Paris (FR)
(74) Mandataire: Sayettat, Julien Christian
(86) Numéro de dépôt international: PCT/FR2015/052150
(87) Numéro de publication internationale: WO 2016/020617

(56) Documents cités:
- US-A- 3 611 965
- US-A- 3 630 198
- US-A- 4 449 973
- US-A- 5 741 284

## Description

L'invention concerne un dispositif pour l'introduction d'une canule à bout arrondi sous la peau d'un patient en vue de l'injection d'un produit biocompatible, un kit d'injection comprenant un tel dispositif et au moins une canule d'injection à bout arrondi, ainsi qu'un procédé d'injection dans le cadre d'une intervention esthétique d'un produit biocompatible au moyen d'un tel kit.

Elle s'applique en particulier aux injections d'un produit de comblement, notamment à base d'acide hyaluronique, sous la peau d'au moins une partie du corps d'un patient, par exemple du visage et/ou du cou dudit patient, afin de faire disparaître temporairement les effets jugés inesthétiques du vieillissement de la peau, comme par exemple les rides et le relâchement de la peau, et/ou de revitaliser ladite peau en hydratant les couches de l'épiderme.

Elle peut également s'appliquer aux injections en autogreffe de produits organiques autologues préalablement prélevés sur le patient, comme du plasma riche en plaquettes (PRP), par exemple pour une stimulation fibroblastique et collagénique de la peau en vue de corriger les effets inesthétiques dus à son vieillissement, ou encore de la graisse, par exemple pour corriger les pertes de volume de la peau dus à un traumatisme tel qu'un accident ou une opération chirurgicale.

Pour pratiquer une telle intervention, le praticien utilise généralement une canule d'injection qui présente un bout arrondi et au moins un orifice latéral d'injection disposé à proximité dudit bout arrondi. En effet, ce type de canule est particulièrement adapté aux produits de comblement, qui sont généralement assez épais, et permet de minimiser les traumatismes sur les tissus et les vaisseaux des couches profondes de la peau, comme les hématomes, les ecchymoses ou les oedèmes, ainsi que les traumatismes nerveux.

Durant l'intervention, le praticien commence par percer, avec une aiguille présentant une pointe distale acérée, une zone de la peau localisée non loin d'une zone à traiter, afin de ménager un accès à ladite zone à traiter.

En particulier, le praticien utilise une aiguille d'injection standard pourvue d'une embase en plastique normalement agencée pour permettre le montage de ladite aiguille sur l'embout d'une seringue, et réalise le perçage de la peau en saisissant ladite embase à deux doigts, ce qui constitue un usage détourné de ladite aiguille.

Ensuite, le praticien retire l'aiguille de perçage et introduit par le trou ainsi ménagé une canule d'injection telle que décrite précédemment, puis fait coulisser sous la peau ladite canule jusqu'à ce que l'orifice d'injection parvienne dans la zone à traiter. Il injecte ensuite le produit dans la zone à traiter par l'intermédiaire de la canule, le produit étant par exemple contenu dans une seringue ou dans un autre type de contenant sur le(la)quel(le) ladite canule est montée.

Cependant, ce type de technique ne donne pas entière satisfaction, en ce qu'elle nécessite un usage en deux temps distincts, dans la mesure où le praticien doit d'abord retirer complètement l'aiguille de la peau avant de pouvoir insérer la canule d'injection. En particulier, les entrées et sorties successives de l'aiguille puis de la canule provoquent des traumatismes sur les tissus cutanés.

En outre, le trou formé par l'aiguille de perçage est parfois difficile à repérer, notamment parce que le praticien doit retirer l'aiguille et la poser sur une surface stérile avant de saisir la canule d'injection, et quitte de ce fait momentanément la zone percée des yeux. Ainsi, le praticien peut perdre du temps à tenter de repérer le trou.

En particulier, pour repérer le trou, le praticien peut être contraint de pincer la peau, parfois plusieurs fois, de sorte à provoquer un saignement par ledit trou, ce qui peut s'avérer douloureux pour le patient et générer des hématomes, voire un gonflement de la zone pincée à fin de saignement. De ce fait, la récupération du patient s'en trouve allongée, et la présence de traces inesthétiques peut présenter un préjudice pour le patient, alors contraint à une éviction sociale temporaire durant ladite récupération.

De plus, même si le praticien repère le trou, il doit aussi mémoriser l'angle d'incidence de l'aiguille. En effet, s'il ne respecte pas le même angle, la canule peut se frayer un autre chemin dans l'épiderme et augmenter ainsi la lésion.

Par ailleurs, une telle technique requiert de nombreuses manipulations de l'aiguille de perçage, notamment pour l'insérer et la retirer de la peau du patient, ce qui augmente le risque de piqûres accidentelles pour le praticien ainsi que le risque de perte de stérilité de ladite aiguille, les risques de piqûres accidentelles étant d'autant plus augmentés que la manipulation de ladite aiguille manque d'ergonomie, dans la mesure où ladite aiguille n'est pas spécialement adaptée pour une telle utilisation.

US 3 630 198, US 3 611 965, US 5741 284 et US 4 449 973 décrivent des dispositifs pour introduire des tubes dans le corps d'un patient, ces dispositifs sont équipés d'une gouttière faisant saillie pour percer la peau d'un patient ainsi que de deux bras latéraux de préhensions.

L'invention vise à perfectionner l'art antérieur en proposant notamment un dispositif pour permettre au praticien de réaliser plus facilement et plus rapidement l'introduction sous la peau d'une canule d'injection, et donc l'injection d'un produit, et ce tout en diminuant de façon importante le risque de traumatismes pour le patient.

A cet effet, selon un premier aspect, l'invention propose un dispositif pour l'introduction d'une canule à bout arrondi sous la peau d'un patient en vue de l'injection d'un produit biocompatible comme dans la revendication 1.

Selon un deuxième aspect, l'invention propose un kit d'injection d'un produit biocompatible, ledit kit comprenant un tel dispositif d'introduction ainsi qu'au moins une canule d'injection à bout arrondi, la gouttière étant agencée pour permettre le coulissement sur elle de ladite au moins une canule au-delà de sa pointe distale vers au moins une zone à traiter située sous la peau d'un patient.

Un procédé d'injection dans le cadre d'une intervention esthétique d'un produit biocompatible au moyen d'un tel kit, ledit procédé prévoyant les étapes successives suivantes de :
- préhension du dispositif par l'intermédiaire de la surface de tenue digitale d'au moins un des bras de préhension ;
- perçage d'une zone de la peau d'un patient au moyen de la pointe distale acérée ;
- disposition d'une canule d'injection sur la gouttière ;
- coulissement de ladite canule d'injection au-delà de la pointe distale acérée, afin d'introduire le bout arrondi sous la peau ;
- retrait du dispositif afin d'extraire la pointe acérée de la peau en laissant la canule introduite sous ladite peau.

Les procédés décrit dans la description ne sont pas revendiqués et sont laissés à des fins illustratives.

D'autres particularités et avantages de l'invention apparaîtront dans la description qui suit, faite en référence aux figures annexées, dans lesquelles :

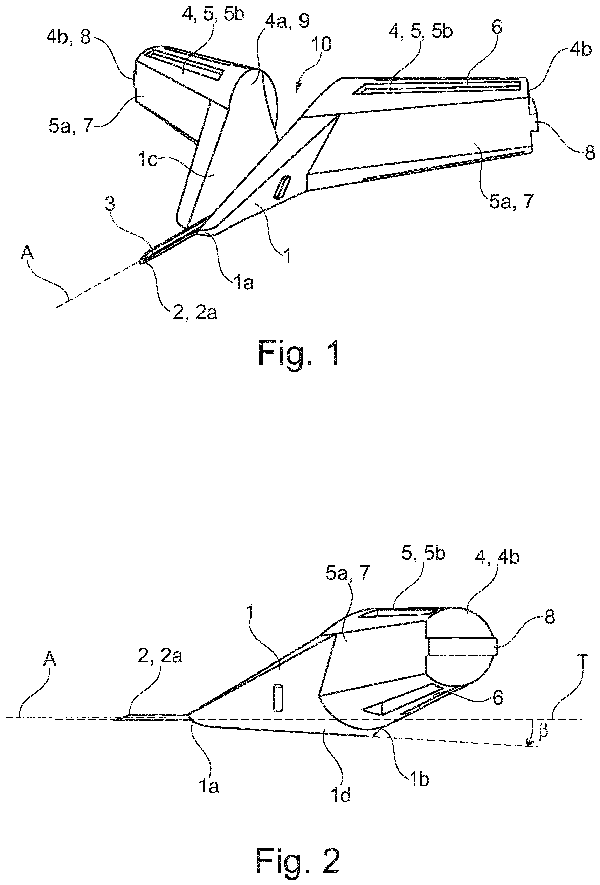

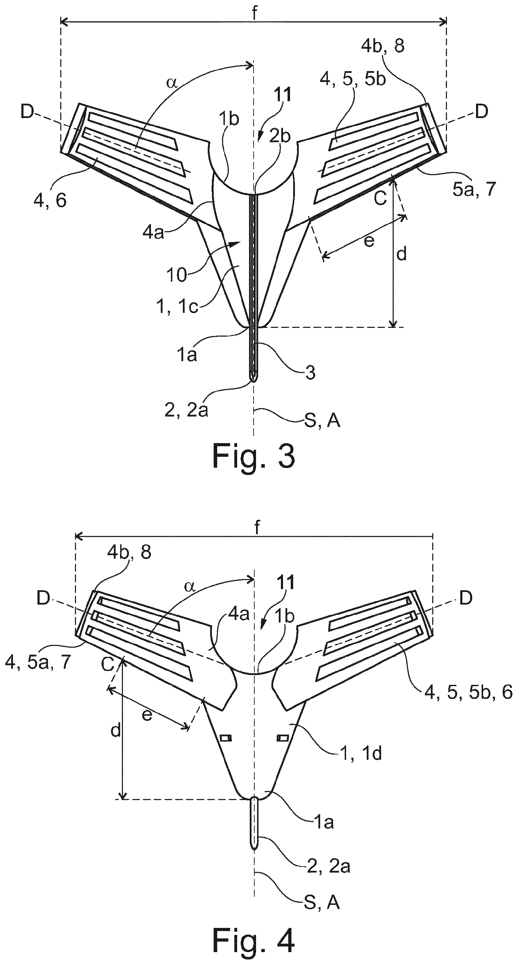

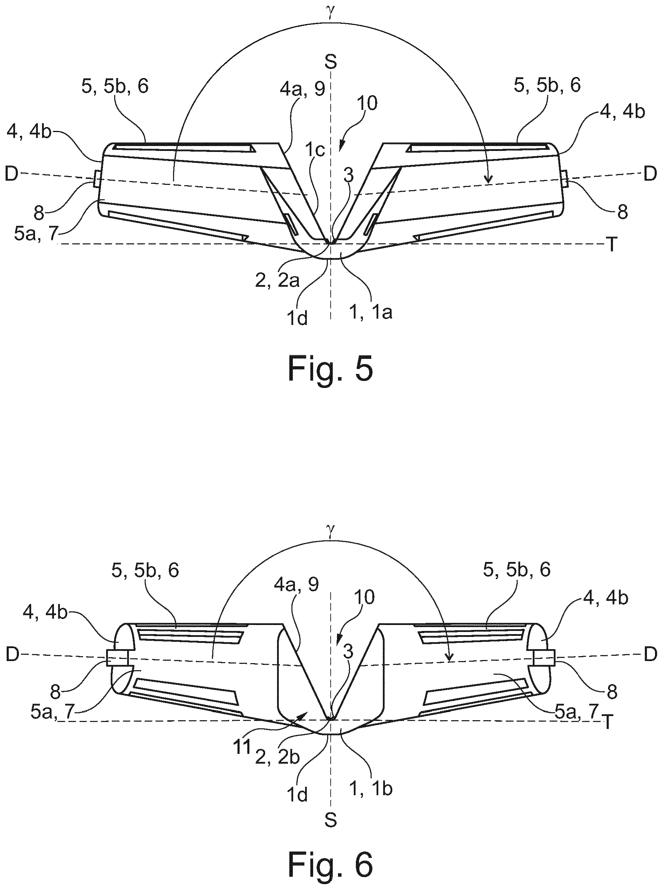

- les figures 1 à 6 représentent un dispositif d'introduction d'une canule selon un mode de réalisation de l'invention, respectivement en perspective avant (figure 1), en vue de côté (figure 2), en vue de dessus (figure 3), en vue de dessous (figure 4), en vue de face (figure 5) et en vue de dos (figure 6) ;

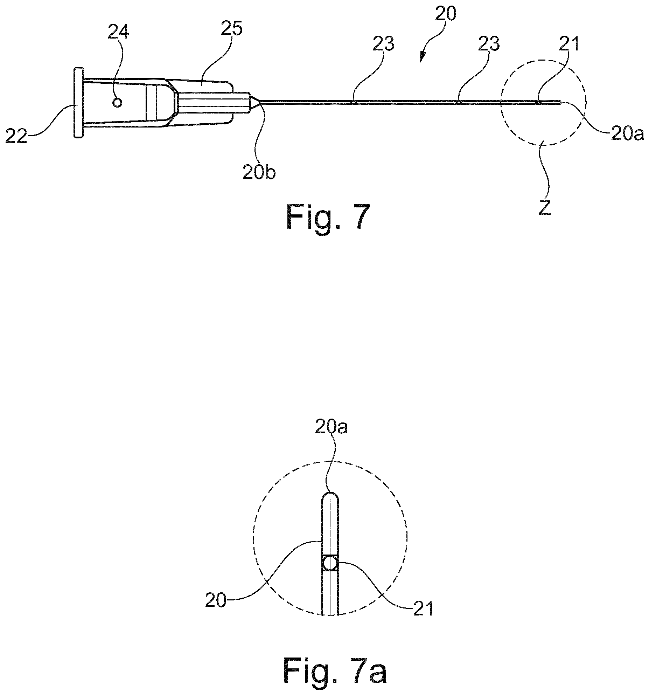

- la figure 7 représente une canule d'injection utilisable dans un kit d'injection avec le dispositif des figures 1 à 6, la figure 7a représentant une vue agrandie de la zone Z de la figure 7 ;

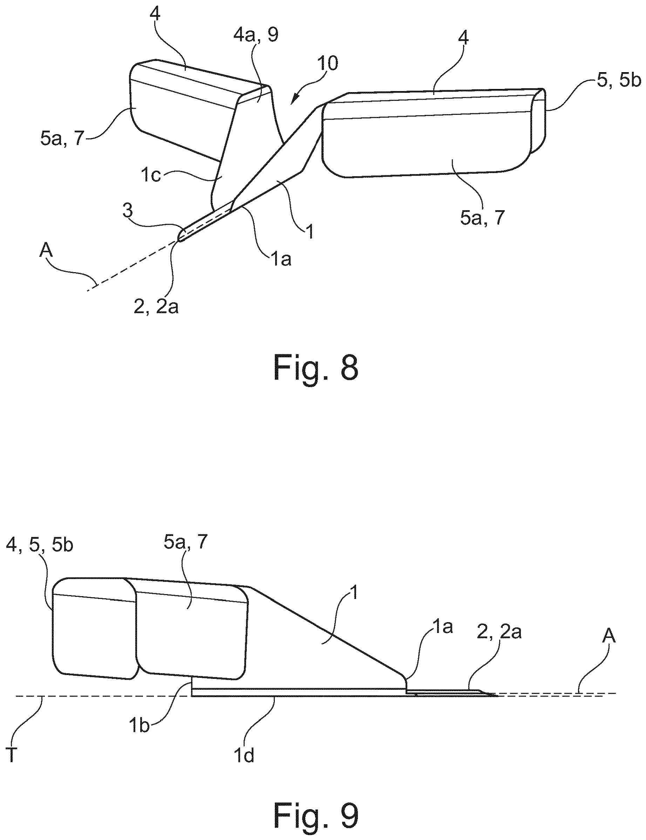

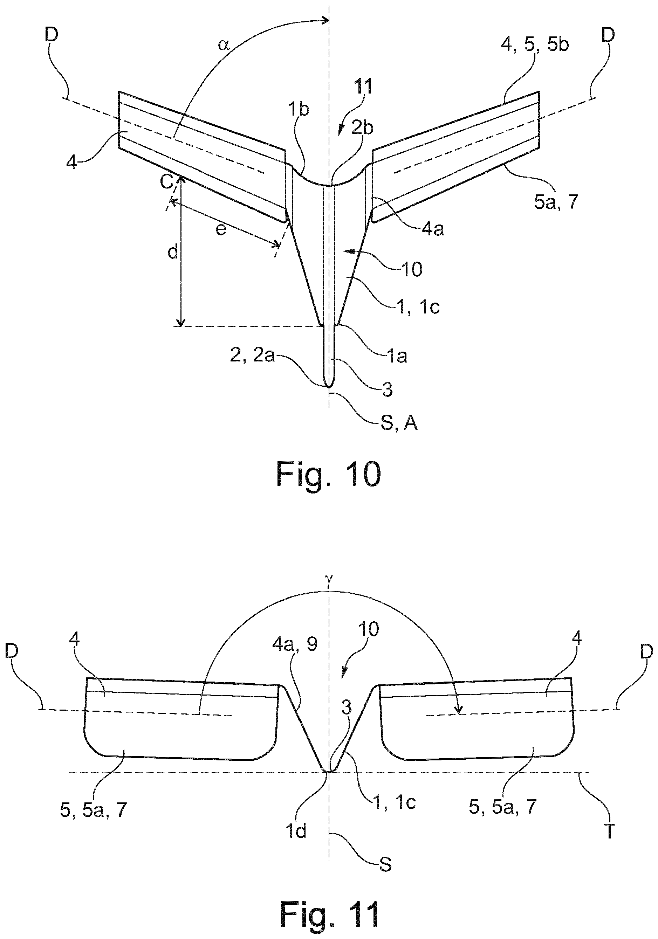

- les figures 8 à 11 représentent un dispositif d'introduction d'une canule selon un autre mode de réalisation de l'invention, respectivement en perspective avant (figure 8), en vue de côté (figure 9), en vue de dessus (figure 10) et en vue de face (figure 11), la canule de la figure 7 étant en outre utilisable avec ledit dispositif.

En relation avec ces figures, on décrit ci-dessous un dispositif pour l'introduction d'une canule à bout arrondi sous la peau d'un patient en vue de l'injection d'un produit biocompatible, un kit d'injection comprenant un tel dispositif et au moins une canule d'injection à bout arrondi, ainsi qu'un procédé d'injection dans le cadre d'une intervention esthétique d'un produit biocompatible au moyen d'un tel kit.

Le kit d'injection peut également être utilisé pour l'injection d'un produit biocompatible dans le cadre d'une intervention médicale ou vétérinaire, ledit produit contenant par exemple principalement un anesthésiant. En particulier, le kit d'injection peut être un dispositif médical à usage unique destiné à être utilisé par du personnel qualifié, notamment du personnel médical ou paramédical.

Dans le cadre d'une intervention esthétique, le produit à injecter peut être un produit de comblement, par exemple à base d'acide hyaluronique, pour corriger temporairement des effets inesthétiques du vieillissement de la peau. En particulier, un produit à base d'acide hyaluronique fluide peut être employé pour lisser la peau afin d'atténuer les rides. De même, un produit à base d'acide hyaluronique réticulé, donc plus épais, peut être employé pour redonner du volume à la peau et/ou pour étirer ladite peau, afin de contrebalancer un relâchement dû à une perte d'élasticité de ladite peau et/ou de corriger une asymétrie, dans le cas où une zone de la peau présente un relâchement et/ou une perte de volume plus importante que sa zone symétrique.

Le produit à injecter peut également comprendre un additif présentant des propriétés anesthésiantes, par exemple à base de lidocaïne, afin de diminuer la douleur que peut ressentir le patient durant l'injection, cette douleur étant d'autant plus importante que ledit produit est épais. Le produit à injecter peut aussi comprendre un additif présentant des propriétés antioxydantes, par exemple à base de mannitol, ainsi que des vitamines pour revitaliser les couches de la peau dans la zone à traiter.

Le produit à injecter peut également être un produit organique autologue préalablement prélevé sur le patient, comme par exemple de la graisse ou du plasma riche en plaquettes (PRP), à visée purement esthétique et/ou afin de permettre des injections en auto greffe, notamment pour corriger des effets inesthétiques dus à un traumatisme tel qu'un accident ou une opération chirurgicale.

La zone à traiter peut être localisée sur le visage ou le cou du patient, notamment sur les parties du visage ou du cou les plus sujettes à des effets inesthétiques de vieillissement de la peau, comme les rides ou un relâchement.

Par exemple, la zone à traiter peut être localisée à une commissure des lèvres (sujette aux « plis d'amertume »), à un coin externe d'un oeil (sujet aux rides en « patte d'oie »), sur une tempe, sur le front, et notamment au niveau de la glabelle (sujette aux « rides du lion »), au niveau d'une zone orbitale (où des cernes et/ou des poches peuvent apparaître), de la vallée des larmes (localisée sous un oeil) d'une pommette ou d'une joue (où un relâchement et/ou une perte volume de la peau peut être constaté(e)).

La zone à traiter peut également être située sur d'autres parties du corps notamment sujettes à de tels effets de vieillissement, comme les mains, mais aussi les bras, les cuisses, la poitrine pour les femmes, notamment au niveau du décolleté, le cuir chevelu, le ventre, ou encore les pieds.

Selon le produit à injecter et l'effet esthétique souhaité, la zone à traiter peut être localisée à des profondeurs diverses de la peau du patient. Par exemple, pour la correction des rides, la zone à traiter est généralement située dans une couche superficielle de la peau, notamment de l'épiderme ou du derme. Au contraire, pour la correction d'un relâchement de la peau, d'un manque de volume, et d'une éventuelle asymétrie résultante, la zone à traiter est plutôt située dans une couche profonde de la peau, notamment de l'hypoderme.

En particulier, la zone à traiter peut être située dans le stratum granulosum, c'est-à-dire la dernière couche de cellules nucléées de l'épiderme. La zone à traiter peut également être située sous l'épiderme, notamment entre le derme et l'os, et plus particulièrement entre le derme profond et le périoste de l'os.

Avant de procéder à l'intervention, le praticien peut déterminer les zones à traiter par un examen minutieux du patient, notamment au moyen d'un éclairage adapté, pour repérer les éventuels manques de volume et les asymétries qui peuvent en résulter, mais aussi pour repérer les vaisseaux sanguins qu'il conviendra d'éviter pour ne pas créer de traumatismes au patient.

Cet examen comprend des observations locales mais aussi une observation globale, notamment s'agissant du visage, afin de corriger les effets inesthétiques de façon harmonieuse et ainsi de garantir un résultat naturel pour le patient. Une fois les zones à traiter identifiées, le praticien peut les marquer au moyen d'un crayon hypoallergénique.

Par ailleurs, pour limiter les risques d'infection, le praticien doit évidemment prendre des mesures d'hygiène, notamment avant de procéder au marquage des zones à traiter. Pour ce faire, le praticien peut nettoyer la peau pour enlever d'éventuelles impuretés, notamment du maquillage, et appliquer une solution antiseptique sur ladite peau.

Pour l'injection d'un produit, le kit comprend au moins une canule 20 à bout arrondi 20a, ladite canule présentant au moins un orifice d'injection 21 qui peut notamment être disposé latéralement à proximité dudit bout arrondi. En particulier, l'orifice 21 présente un diamètre compris entre 0,05 mm et 1 mm et est localisé à environ 2 mm du bout arrondi 20a.

De façon connue, l'utilisation de canules d'injection à bout arrondi s'avère particulièrement avantageuse dans le cadre d'une intervention esthétique, en ce qu'elles permettent non seulement d'éviter la section de vaisseaux sanguins et des traumatismes nerveux lors de leur introduction sous la peau d'un patient, et ainsi de limiter les traumatismes pour ledit patient, mais aussi de limiter le risque de piqûre accidentelle pour le praticien.

Selon le produit à injecter, la profondeur de la zone à traiter et/ou sa localisation par rapport au trou d'accès, les dimensions de la canule 20 peuvent varier. En particulier, la canule 20 peut présenter un calibre externe réduit si le produit est fluide et au contraire plus important si le produit est épais. Par exemple, la canule 20 peut présenter un calibre externe compris entre 14G (14 gauges, ce qui correspond à un diamètre de 2 mm) et 33G (0,2 mm), et notamment entre 22G (0,7 mm) et 25G (0,5 mm).

La canule 20 peut également présenter un calibre interne plus ou moins important, notamment choisi parmi trois tailles standard (normal, large (thin wall en anglais), extra-large (extra-thin wall en anglais), ledit calibre interne ayant notamment une influence sur la pression appliquée au produit durant l'injection. Ainsi, avec un calibre interne large, le praticien peut injecter un produit épais sans appliquer de pression trop importante audit produit, ce qui permet de faciliter ladite injection et de réduire la douleur pour le patient. Par ailleurs, en appliquant moins de pression, les molécules du produit sont moins soumises à des contraintes physiques et donc moins susceptibles d'être détériorées.

Par ailleurs, la canule 20 peut présenter une longueur variable, selon la profondeur et/ou la localisation de la zone à traiter avec ladite canule par rapport au trou d'accès. Cette longueur peut notamment être comprise entre 13 mm et 110 mm, et plus particulièrement comprise entre 25 mm et 70 mm.

La canule 20 est avantageusement flexible, afin de faciliter son guidage vers la zone à traiter, puis le positionnement de son orifice d'injection 21 dans ladite zone à traiter. En particulier, la flexibilité de la canule 20 est liée au calibre externe, et notamment au rapport entre ledit calibre externe et le calibre interne, ladite flexibilité étant d'autant plus importante que ledit rapport, et donc l'épaisseur de la paroi de la canule 20, est réduit(e).

En outre, la canule 20 peut être au moins partiellement réalisée en acier inoxydable, notamment en alliage SUS 304 ou en alliage AISI 304 (de formule chimique brute FeCr₁₈Ni₁₀), ce dernier étant particulièrement remarquable pour ses bonnes performances en termes de flexibilité, de robustesse, d'élasticité, de résistance à la corrosion et de tolérance par l'organisme du patient, et/ou en un alliage à mémoire de forme.

De plus, la surface extérieure de la canule 20 peut avoir subi un traitement spécial, par exemple à base de silicone, afin de faciliter le coulissement de ladite canule sous la peau du patient, et donc de minimiser les traumatismes pour ledit patient, notamment les hématomes, les ecchymoses ou les oedèmes.

De façon connue, pour injecter le produit de traitement, la canule 20 est montée sur une seringue ou un autre type de contenant dans lequel une quantité dudit produit est conditionnée.

Pour ce faire, la canule 20 présente une embase 22 sur laquelle une extrémité proximale 20b de ladite canule opposée au bout arrondi 20a est montée, par exemple en étant fixée au moyen d'un adhésif dans un logement prévu à cet effet sur ladite embase, ladite embase étant destinée à être montée sur un embout d'une seringue contenant le produit à injecter.

Par exemple, l'embase 22 peut être de type Luer-Lock^{®} ou Luer-Slip^{®}, pour être montée respectivement par vissage ou par friction sur l'embout de la seringue, ces types étant avantageusement universels pour permettre l'utilisation de la canule 20 avec tous types de seringues ou d'autres contenants. En particulier, l'embase 22 comprend des ailettes 25 pour permettre de se connecter au contenant type seringue. En variante, l'embase 22 peut présenter un pas de vis.

Par ailleurs, l'embase 20 peut être réalisée en polypropylène ou en un copolymère de polypropylène, notamment l'éthylène-polypropylène. De façon générale, l'embase 20 peut être réalisée en un matériau ininflammable, notamment dépourvu de pyrogène.

Pour permettre au praticien d'identifier rapidement le calibre externe et/ou le calibre interne de la canule 20, et ainsi sélectionner ladite canule en fonction de ses besoins, l'embase 22 de ladite canule peut comprendre un marquage d'identification de son calibre externe et/ou de son calibre interne.

En particulier, le marquage d'identification consiste en une coloration de l'embase 22, la coloration correspondant au calibre interne et/ou au calibre externe de la canule 20, comme défini notamment par la norme NF EN ISO 6009.

Pour pouvoir introduire une telle canule 20 sous la peau d'un patient, le procédé prévoit une étape de perçage d'une zone de ladite peau, afin de ménager pour la canule 20 un trou d'accès sous ladite peau.

Pour ce faire, le kit d'injection comprend un dispositif pour l'introduction de la canule 20 sous la peau du patient, ledit dispositif comprenant un corps central 1 équipé d'une gouttière 2 qui présente une pointe distale acérée 2a faisant saillie depuis une extrémité distale 1a dudit corps pour le perçage de la peau.

En relation avec les figures, la gouttière 2 s'étend suivant un plan sagittal S qui forme un plan de symétrie pour le dispositif, et notamment pour le corps central 1, le dispositif étant représenté suivant ledit plan sagittal sur la figure 2.

La gouttière 2 définit en outre :
- un plan transversal T qui est perpendiculaire au plan sagittal S et sur lequel la gouttière 2 s'étend jusqu'à sa pointe distale 2a, ledit plan transversal passant notamment dans ladite pointe distale, le dispositif étant représenté suivant ledit plan transversal sur les figures 3, 4 ; et
- un plan frontal qui s'étend perpendiculairement au plan sagittal S, le dispositif étant représenté suivant ledit plan frontal sur les figures 5, 6.

En particulier, la longueur de la pointe distale acérée 2a est limitée pour assurer une pénétration de ladite pointe sur une profondeur limitée de la peau du patient, et ainsi éviter les traumatismes sur les tissus et les vaisseaux des couches profondes de la peau, comme les hématomes, les ecchymoses ou les oedèmes, ainsi que les traumatismes nerveux.

L'extrémité distale 1a du corps central 1 présente une forme émoussée et notamment arrondie, ainsi qu'une forme évasée dans le plan transversal T, afin d'assurer un contact non traumatisant de ladite extrémité distale contre ladite peau lorsque la pointe acérée 2a est entièrement insérée dans ladite peau, un tel contact présentant en outre l'avantage d'être rassurant psychologiquement pour ledit patient.

La zone à percer peut notamment être localisée sur le visage du patient, par exemple près d'une oreille (point rétro-mandibulaire), dans une zone naso labiale (au niveau du modiolus, point naso-génien), au niveau de l'os zygomatique (point zygomatique), sur une tempe (point temporal ou frontal) ou au-dessus de la glabelle (point glabellaire). Ainsi, le praticien peut atteindre plusieurs zones à traiter localisées sur le visage et/ou le cou du patient à partir d'un même trou ménagé dans ladite zone à percer, ce qui permet de limiter les perçages de la peau, et donc les traumatismes pour le patient. En particulier, pour une intervention sur le visage, le nombre de zones à percer peut être compris entre une et cinq pour chaque côté dudit visage.

La zone à percer peut être également localisée sur toute autre partie du corps du patient, notamment sur une main, un bras ou une cuisse, sur la poitrine, notamment au niveau du décolleté, sur le cuir chevelu, sur le ventre, ou encore sur un pied.

Pour percer la peau du patient, le praticien, après avoir préparé ladite peau, saisit le dispositif avec ses doigts et insère la pointe distale acérée 2a dans ladite peau. Une fois la peau du patient percée, le procédé prévoit de disposer la canule 20 d'injection sur la gouttière 2, la pointe acérée 2a du dispositif restant plantée dans la peau. Ensuite, le procédé prévoit une étape consécutive de coulissement de la canule d'injection 20 au-delà de la pointe distale acérée 2a, afin d'introduire le bout arrondi 20a sous la peau.

Pour ce faire, la gouttière 2 est agencée pour permettre le coulissement sur elle de la canule d'injection 20 au-delà de sa pointe distale 2a vers au moins une zone à traiter située sous la peau du patient. En particulier, la gouttière 2 présente une dimension interne qui est supérieure au calibre externe de la canule d'injection 20 devant coulisser sur elle.

En relation avec les figures, la gouttière 2 présente une extrémité proximale 2b ouverte qui débouche dans une paroi proximale 1b du corps central 1. Ainsi, de façon particulièrement intuitive, le procédé peut prévoir de disposer la canule 20 sur la gouttière 2 en introduisant le bout arrondi 20a de ladite canule dans l'extrémité proximale 2b, puis de faire coulisser ledit bout arrondi sur toute la longueur de la gouttière 2.

Par ailleurs, la gouttière 2 présente une fente supérieure 3 qui est entourée par une paroi supérieure 1c du corps central, ladite fente s'étendant sur toute la longueur de ladite gouttière.

En particulier, la fente 3 peut présenter un écartement supérieur au calibre externe de la canule 20. Ainsi, pour pouvoir faire coulisser la canule 20 sur la gouttière 2, le praticien peut disposer le bout arrondi 20a sur n'importe quel point de ladite gouttière en introduisant ledit bout arrondi latéralement par la fente 3, ce qui facilite ladite disposition et permet au praticien de gagner du temps.

En variante, la fente 3 peut présenter un écartement inférieur au calibre externe de la canule 20, de sorte que ladite canule ne peut être extraite latéralement de la gouttière 2 par ladite fente. Dans ce cas, pour disposer la canule 20 sur la gouttière 2, le praticien ne peut introduire le bout arrondi 20a de ladite canule que dans l'extrémité proximale 2b de ladite gouttière.

Dans le mode de réalisation représenté, la gouttière 2 présente une géométrie d'une section longitudinale de cylindre, notamment une géométrie hémicylindrique réalisée au moyen d'une demi-aiguille coupée longitudinalement sur toute ou une partie de sa longueur. Ainsi, la gouttière 2 présente une géométrie de forme complémentaire à celle de la canule d'injection 20, ce qui permet de faciliter le coulissement de ladite canule sur ladite gouttière.

En particulier, le diamètre interne de la gouttière 2 dépend de la proportion de diamètre à laquelle la section de cylindre est effectuée, ledit diamètre étant d'autant plus important que ladite proportion est faible. Ainsi, lorsque la gouttière 2 présente une géométrie hémicylindrique, son diamètre interne correspond à l'écartement de la fente 3 et est supérieur au calibre externe de la canule 20, ce qui permet de disposer ladite canule sur ladite gouttière en introduisant le bout arrondi 20a sur n'importe quel point de ladite gouttière.

Ainsi, le praticien peut gagner du temps par rapport aux techniques d'injection classiques, car il n'a pas à localiser le trou formé par perçage, puisque le dispositif ayant servi audit perçage reste positionné dans ledit trou et que l'accès à la zone à traiter est matérialisé par la gouttière 2, qui est facilement localisable. De ce fait, le praticien n'a donc pas besoin de recourir à des pratiques traumatisantes pour le patient, comme de pincer plusieurs fois la peau dudit patient pour repérer le trou en risquant de la faire saigner.

En outre, grâce au guidage de la canule 20 par la gouttière 2, le praticien tâtonne moins durant l'introduction de ladite canule, ce qui s'avère également moins traumatique pour le patient.

De plus, les opérations de manipulation du dispositif de perçage sont limitées, ce qui permet de limiter les risques de piqûre accidentelle pour le praticien et le risque de perte de stérilité de la pointe acérée 2a dudit dispositif.

Selon la profondeur de la zone à traiter et/ou sa localisation par rapport au trou d'accès, la canule 20 peut être introduite sous la peau suivant des directions angulaires variées par rapport à ladite peau. En particulier, la canule 20 peut être introduite suivant une direction sensiblement tangente à la surface de ladite peau, notamment si le produit doit être injecté dans une seule zone ou dans plusieurs zones sensiblement localisées sur un même chemin de progression. Au contraire, la canule 20 peut être introduite suivant une direction sensiblement perpendiculaire à la surface de la peau, notamment si le produit doit être injecté dans plusieurs zones localisées dans des directions nettement différentes. En outre, l'épaisseur de la peau et/ou sa texture peuvent également influencer l'angle d'introduction de la canule 20.

Par conséquent, pour guider correctement la canule 20 vers la(les) zone(s) à traiter, la gouttière 2, et donc le dispositif, doivent être orientés suivant la direction angulaire d'introduction de ladite canule lors de l'insertion de la pointe acérée 2a dans la peau, le praticien devant en outre maintenir ledit dispositif suivant ladite orientation au moins jusqu'à ce que le bout arrondi 20a de ladite canule soit introduit sous ladite peau.

Pour ce faire, le corps central 1 est pourvu de deux bras latéraux 4 de préhension de forme allongée qui s'étendent symétriquement de part et d'autre du plan sagittal S, chaque bras de préhension 4 présentant une surface 5 de tenue digitale.

En particulier, la forme allongée des bras 4 de préhension et leur disposition latérale de part et d'autre de la gouttière 2 permet au praticien de saisir facilement le dispositif en tenant un seul desdits bras par sa surface 5 de tenue digitale, notamment sur une portion centrale avant 5a et sur une portion arrière 5b opposée de ladite surface, par exemple en pinçant ledit bras entre son pouce et son index.

Ainsi, la main du praticien qui tient le dispositif peut être facilement disposée à l'écart de l'axe A de la gouttière 2 et du plan sagittal S, et donc de l'axe de guidage de la canule 20, et ce sans contraindre ledit praticien à adopter avec ladite main une position inconfortable et potentiellement douloureuse à long terme pour son poignet. De ce fait, le praticien peut facilement disposer et faire coulisser la canule 20 sur la gouttière 2 en utilisant son autre main, sans que ses deux mains ne se gênent mutuellement.

En relation avec les figures, notamment les figures 3 et 4, les bras latéraux 4 s'étendent chacun suivant une direction transversale D qui forme avec le plan sagittal S un angle α compris entre 30° et 85°, et notamment de 65°.

Cette plage angulaire offre un bon compromis pour à la fois faciliter le maintien du dispositif par préhension bi-digitale d'un de ses bras 4 et limiter l'encombrement de l'axe de la gouttière 2 par la main maintenant ledit dispositif.

En effet, pour un maintien optimal du dispositif par préhension bi-digitale, la direction transversale D des bras 4 ne doit pas être trop écartée du plan sagittal S, afin de ne pas trop écarter l'axe de l'effort à fournir pour piquer la peau par rapport à l'axe A de la gouttière 2. En particulier, lorsque l'angle α est supérieur à 85°, le dispositif est inadapté à un maintien par préhension bi-digitale, car un tel maintien génère un effet de porte à faux qui est gênant pour le perçage de la peau.

Par ailleurs, plus l'angle α est faible, plus l'effort de piquage de la peau est proche de l'axe A, mais en contrepartie la main encombre davantage l'axe A de la gouttière 2, ce qui s'avère gênant pour le guidage de la canule 20. En particulier, lorsque l'angle α est inférieur à 30°, l'ergonomie du dispositif est compromise en ce que les mains du praticien se gênent mutuellement, ce qui contraint ledit praticien à adopter avec la main tenant le dispositif une posture inconfortable et potentiellement douloureuse, par exemple en disposant ladite main sensiblement à plat par rapport à la peau du patient et suivant un angle peu naturel par rapport à son poignet.

De façon avantageuse, l'angle α est compris entre 45° et 80°, et plus particulièrement entre 65° et 80°, ce qui confère au dispositif une ergonomie optimale.

En outre, pour un angle α compris entre 30° et 40°, la forme des bras 4 peut être adaptée pour libérer davantage l'axe A de guidage de la canule. En particulier, les bras 4 peuvent présenter une forme en courbe suivant leur direction transversale D, le rayon de courbure de ladite forme étant en outre suffisamment important, notamment supérieur à 20 mm, pour faciliter la préhension des bras 4.

Par ailleurs, chaque bras 4 de préhension présente une extrémité proximale 4a solidarisée à proximité de la paroi proximale 1b du corps central 1, la portion centrale avant 5a de chaque bras 4 présentant un centre C qui s'étend à une distance e d'environ 10 mm de ladite extrémité proximale, ledit centre correspondant à l'épicentre de la pression exercée par le doigt lorsque le praticien saisit le bras 4. On notera que la longueur de chaque bras 4 n'influe pas significativement sur l'ergonomie et n'influe donc pas significativement sur la position de l'épicentre C.

En particulier, la portion centrale avant 5a de chaque surface 5 de tenue digitale est située, notamment au niveau de son épicentre C, à une distance d comprise entre 8 mm et 30 mm de l'extrémité distale 1a du corps central 1, une telle plage de valeurs correspondant aux épaisseurs moyennes d'un doigt.

Ainsi, lorsque le praticien tient le dispositif en plaçant son index sur la portion centrale avant 5a de la surface 5 de tenue digitale d'un bras 4 et son pouce sur la portion arrière 5b de ladite surface, puis pique la peau en maintenant ainsi ledit dispositif, son index vient se placer entre la peau et ledit bras, notamment lorsque la pointe acérée 20a est insérée suivant un angle sensiblement perpendiculaire à ladite peau, et ce sans gêner ladite insertion. Par ailleurs, si la distance d est suffisamment petite, l'index peut se trouver au plus près de la peau, et notamment en appui entre le bras 4 de préhension et ladite peau, ce qui confère une meilleure stabilité au dispositif et facilite son maintien durant l'introduction de la canule 20 sous la peau.

En particulier, la distance d est comprise entre 10 et 25 mm, et notamment égale à 13 mm, ce qui confère au dispositif une ergonomie optimale. Lorsque la distance d se situe en deçà de 8 mm, le dispositif est inadapté à une préhension bi-digitale d'un bras 4, en ce que le doigt en appui sur la portion centrale avant 5a gênerait pour le perçage de la peau. Par ailleurs, une distance d située au-delà de 30 mm ne permet pas au praticien de reposer son doigt contre la peau lors du perçage, et a donc un impact négatif sur la précision dudit perçage.

En relation avec les figures 1 à 6, pour faciliter leur préhension, les bras 4 présentent chacun une géométrie volumique, et notamment une géométrie essentiellement cylindrique autour de leur direction transversale D. Ainsi, le praticien peut saisir le dispositif de façon variée, selon ses préférences, sa position corporelle par rapport au patient, la zone à percer et/ou la direction angulaire suivant laquelle la canule 20 doit être introduite.

En variante, comme représenté sur les figures 8 à 11, les bras 4 peuvent présenter une géométrie avec des formes plus plates, notamment une géométrie rectangulaire, afin de faciliter la préhension bi-digitale desdits bras.

En particulier, les bras 4 présentent une géométrie légèrement tronconique, ce qui permet une posture détendue et précise de l'index et du pouce du praticien pour saisir lesdits bras en préhension bi-digitale, et ce que ledit praticien soit gaucher ou droitier. Par ailleurs, le praticien peut, par de petites rotations, positionner par tâtonnements successifs la pointe distale acérée 2a et ajuster sa direction angulaire d'insertion sous la peau.

Pour faciliter ces petites rotations, notamment en empêchant les doigts du praticien de glisser sur les surfaces 5 de tenue digitale, lesdites surfaces présentent chacune au moins une portion sur laquelle s'étend une succession de motifs en relief 6 d'aide à la préhension digitale. Sur les figures 1 à 6, les motifs 6 s'étendent suivant la direction transversale D d'un bras 4. En variante, les motifs 6 peuvent présenter une autre configuration, notamment en s'étendant suivant une autre direction, par exemple perpendiculaire à la direction transversale D.

En relation avec les figures 1 à 6, chaque surface 5 de tenue digitale présente un méplat avant 7, sur laquelle est formée la portion centrale avant 5a, ainsi qu'une succession de motifs en relief 6 qui s'étendent sur la portion arrière 5b opposée audit méplat avant. Ainsi, le praticien peut saisir un bras 4 en plaçant son index sur le méplat avant 7, ce qui confère une bonne stabilité audit index, et son pouce sur la portion arrière 5b, afin d'effectuer des petites rotations uniquement au moyen dudit pouce, et ainsi de positionner la pointe acérée 2a de façon précise.

Par ailleurs, chaque bras 4 de préhension présente une extrémité distale 4b sur laquelle est formé un motif en relief 8 d'aide à la tenue digitale. Ainsi, le praticien peut également tenir le dispositif en pinçant les extrémités distales 4b de chaque bras 4 avec respectivement son pouce et son index, les motifs en relief 8 permettant le maintien sans pivotement dudit dispositif entre lesdits doigts.

En particulier, les motifs 8 peuvent se présenter sous la forme d'un rectangle passant par le centre de respectivement une extrémité distale 4b, comme représenté sur les figures, mais aussi sous d'autres formes, telles que par exemple un plot excentré ou deux pans coupés.

En outre, les extrémités distales 4b de chaque bras 4 sont écartées l'une de l'autre suivant une distance f d'environ 34 mm dans le plan transversal T, afin de permettre au praticien de pincer lesdites extrémités avec un écart correct entre ses doigts, c'est-à-dire un écart ni trop grand, pour ne pas générer de fatigue à long terme, ni trop petit, pour ne pas gêner le guidage et l'introduction de la canule 20 sous la peau.

En relation avec la figure 2, le corps central 1 présente une partie inférieure 1d qui s'étend sous le plan transversal T en formant avec ledit plan transversal, et donc avec la pointe acérée 2a, un angle β. Ainsi, en limitant l'étendue de matière du corps 1 sous l'axe A de la gouttière 2, et donc sous la pointe acérée 2a, le dispositif peut être orienté de manière à insérer la pointe acérée 2a suivant une direction tangentielle à la surface de la peau, afin de pouvoir introduire une canule 20 suivant une telle direction.

L'angle β peut présenter une valeur très faible, voire quasiment nulle, comme représenté sur les figures 8 à 11. Toutefois, il arrive que le praticien souhaite faire glisser le dispositif le long de la peau préalablement au perçage, afin par exemple de réfléchir à la localisation précise dudit perçage, et un angle β trop faible rendrait cette manipulation plus délicate, en ce que le praticien pourrait par inadvertance percer la peau à un endroit inapproprié.

L'angle β peut également présenter une valeur plus importante, mais ne doit pas dépasser 15°, sous peine de rendre le dispositif inadapté à un perçage tangentiel de la peau.

En particulier, pour un angle β compris entre 0 et 6°, et notamment égal à 3°, le dispositif présente une ergonomie optimale. Par ailleurs, s'agissant de la possibilité de perçage tangentiel, elle est généralement correcte pour un angle β inférieur ou égal à 12°.

Pour améliorer l'accès à la gouttière 2, et donc faciliter la disposition de la canule 20 sur ladite gouttière, la paroi supérieure 1c du corps 1 présente une zone de dégagement 10 qui s'étend de part et d'autre de ladite gouttière.

En relation avec les figures, le corps central 1 présente une géométrie en forme de V, la paroi supérieure 1c formant ainsi un sillon évasé au centre duquel la gouttière 2 est disposée. Par ailleurs les extrémités proximales 4a des bras 4 de préhension présentent chacune une pente 9 qui s'étend dans le prolongement de respectivement un pan de la paroi supérieure 1c, de sorte que lesdites extrémités proximales forment avec ladite paroi supérieure la zone de dégagement supérieure 10 d'accès à la gouttière 2.

En outre, les bras 4 de préhension s'étendent l'un par rapport à l'autre dans le plan frontal suivant un angle γ compris entre 90° et 180°, et notamment de 172°, une telle plage angulaire permettant d'assurer de façon satisfaisante l'accès à la gouttière 2 par la zone de dégagement supérieure 10.

De même, la paroi proximale 1b du corps central 1 est entourée d'une zone de dégagement arrière 11 agencée pour permettre l'utilisation d'une canule d'injection 20 de faible longueur, notamment en évitant les risques de collision entre la paroi proximale 1b du corps 1 et de l'embase 22 de ladite canule lorsque celle-ci est coulissée sur la gouttière 2, puis introduite sous la peau du patient.

En relation avec les figures, la zone de dégagement arrière 11 présente une forme en arc de cercle et s'étend de part et d'autre de la paroi proximale 1b du corps 1, notamment en s'étendant sur les extrémités proximales 4a des bras 4 de préhension. En particulier, la zone de dégagement arrière 11 présente un rayon de courbure de l'ordre de 5 mm.

Une fois la canule 20 introduite sous la peau, le procédé prévoit de retirer le dispositif afin d'extraire sa pointe acérée 2a de ladite peau, et ce tout en laissant la canule 20 introduite sous ladite peau. En particulier, le procédé peut prévoir de retirer le dispositif par coulissement de la gouttière 2 par rapport à la canule 20 introduite sous la peau.

Après avoir retiré le dispositif, le praticien dispose de ses deux mains pour finir de positionner la canule 20, notamment en faisant coulisser ladite canule sous la peau jusqu'à ce que son orifice d'injection 21 parvienne dans une zone à traiter, afin de pouvoir injecter le produit dans ladite zone.

Pour faciliter le positionnement de l'orifice d'injection 21 dans la zone à traiter, notamment lorsque celle-ci est localisée à une profondeur importante et/ou à une distance importante du trou d'accès, la canule 20 peut présenter des graduations 23 ménagées à des intervalles réguliers sur sa longueur, par exemple tous les centimètres.

Par ailleurs, l'embase 22 de la canule 20 peut comprendre sur sa périphérie un marquage 24 de repérage de l'orifice d'injection 21 qui est aligné avec ledit orifice d'injection, ce qui permet au praticien d'orienter correctement ledit orifice d'injection lorsque celui-ci est positionné dans la zone à traiter.

En relation avec la figure 7, le marquage de repérage comprend un point 24 ménagé sur la périphérie de l'embase 22, ledit point étant réalisé en une couleur suffisamment voyante pour contraster avec la couleur de ladite embase.

En variante, le marquage de repérage 24 peut présenter un autre motif, notamment en forme de flèche. Il peut en outre être réalisé en relief sur l'embase 22, par exemple lors du moulage de ladite embase, et éventuellement coloré avec une teinte différente de ladite embase.

Pour limiter la douleur ressentie par le patient durant l'intervention, le procédé peut prévoir une étape préalable au perçage de la peau, dans laquelle un produit anesthésiant est appliqué au moins sur et/ou dans la zone à percer, ce qui permet de limiter à la fois la douleur due au dit perçage et la douleur due à l'introduction de la canule 20. En particulier, le produit anesthésiant peut être appliqué localement sous forme de crème et/ou de patch, par exemple un patch de type Emla^{®}.

Par ailleurs, le procédé peut prévoir de traiter successivement plusieurs zones, et ce en y accédant à partir du même trou d'accès. En particulier, pour l'injection d'un même produit dans plusieurs zones à traiter, le procédé peut prévoir de déplacer une canule 20 déjà introduite dans la peau du patient vers une autre zone à traiter après injection dudit produit dans une première zone.

En particulier, le procédé peut prévoir un déplacement radial entre la zone qui vient d'être traitée et une nouvelle zone à traiter pour procéder à une nouvelle injection dans ladite nouvelle zone.

En outre, le procédé peut prévoir de répéter au moins une fois un tel déplacement radial depuis la zone traitée vers une autre zone à traiter et une étape consécutive d'injection de produit dans ladite autre zone. Ainsi, le praticien peut traiter toute une zone du visage, par exemple le front, une tempe ou une joue, et ce à partir d'un seul trou d'accès.

Pour corriger un relâchement de la peau largement étendu sur une zone du visage, par exemple sur le front, sur une tempe ou sur une joue, une technique connue consiste à injecter du produit par bolus, c'est à dire à déposer plusieurs petites doses de produit sur l'ensemble de ladite zone en effectuant plusieurs déplacements dans ladite zone, afin d'étirer ladite peau sur toute la zone tout en lui redonnant du volume.

En particulier, le praticien peut procéder à plusieurs injections par bolus en effectuant plusieurs déplacements radiaux, notamment en éventail, dans une même zone, puis massage. Le praticien peut également utiliser une technique particulière appelée « nappage » qui est généralement réalisée dans une couche de l'hypoderme, afin de réaliser un lissage en surface et de repulper la peau en profondeur.

En déplaçant radialement la canule 20 déjà insérée dans la couche hypodermique depuis une zone déjà traitée de ladite couche vers une autre zone à traiter, ladite canule reste notamment à une profondeur constante. Ainsi, le praticien s'assure de réaliser toutes les injections de nappage dans une même couche, et notamment à une même profondeur dans ladite couche.

De ce fait, le praticien peut facilement repulper une couche sous la peau avec une épaisseur constante de produit, ce qui permet de garantir l'homogénéité et l'harmonie du résultat final.

Par ailleurs, le procédé peut prévoir de déplacer la canule 20 en arrière pendant l'injection par bolus du produit, c'est-à-dire suivant un mouvement rétrograde, afin de répartir ledit produit sur un intervalle de profondeurs important et/ou sur une zone plus large, ce qui permet un résultat plus harmonieux et donc plus naturel. En variante, le procédé peut prévoir de déplacer la canule 20 en avant, suivant un mouvement antégrade, durant l'injection par bolus du produit.

Une fois les injections terminées, le praticien retire la canule 20 et peut effectuer un massage sur la zone traitée, afin de répartir uniformément le produit et donner un aspect plus homogène et donc plus naturel à la correction apportée. Le patient peut alors constater des résultats immédiatement visibles et durables.

En relation avec les figures 8 à 11, la gouttière 2 est formée en une seule pièce avec le corps 1 à partir d'une feuille de matériau adapté, notamment en métal, et plus particulièrement en acier inoxydable tel que l'alliage SUS 304 et/ou l'alliage AISI 304. Pour ce faire, une forme adaptée peut être découpée dans une telle feuille, puis formée en trois dimensions par emboutissage de ladite feuille découpée.

En particulier, l'emboutissage permet de générer par fluage des épaisseurs de matière variables, notamment une épaisseur de matière faible au niveau de la gouttière 2 et une épaisseur de matière plus importante au niveau du corps central 1 et des bras 4.

En outre, l'emboutissage peut être complété par des étirements de matière, afin d'obtenir un dispositif avec des formes en trois dimensions plus riches. Ainsi, on peut par exemple relier les reliefs des bras 4 aux reliefs du corps central 1 et obtenir une meilleure rigidité du dispositif dans son ensemble tout en restant dans des épaisseurs fines.

En particulier, la gouttière 2 peut bénéficier d'un affutage après l'emboutissage pour former et/ou aiguiser la pointe distale acérée 2a.

En relation avec les figures 1 à 6, la gouttière 2 et le corps 1 peuvent être formés en une seule pièce par injection d'une matière adaptée, notamment une matière plastique. En particulier, la matière plastique peut comprendre un polymère inoffensif pour le corps humain ou animal, notamment en étant implantable, par exemple en polypropylène ou en un copolymère à base de polypropylène, comme l'éthylène-polypropylène. De façon générale, la gouttière 2 et le corps 1 peuvent être réalisés en un matériau ininflammable, notamment dépourvu de pyrogène.

La gouttière 2 peut également être formée en une pièce distincte rapportée sur le corps 1, par exemple par collage de ladite gouttière sur ledit corps ou par surmoulage de ladite gouttière par ledit corps.

Pour faciliter le positionnement fiable de la gouttière 2 dans le corps 1 lors du collage, on peut prévoir une surépaisseur, préférentiellement de 3 à 5 mm de longueur, dans la partie proximale de la paroi supérieure 1c formant un sillon évasé. Cette surépaisseur peut être percée d'un trou apte à recevoir la gouttière 2, afin que celle-ci soit retenue et positionnée de manière contrainte et fiable lors du collage. La surépaisseur doit être de faible épaisseur, préférentiellement inférieure à 15/1 0èmes de millimètres afin qu'elle ne gêne pas lorsque le praticien introduit la canule dans le trou réalisé par le dispositif.

Pour faciliter la solidarisation de la gouttière 2 au corps 1, l'extrémité proximale 2b de ladite gouttière peut être initialement solidarisée à un élément tubulaire au moyen d'un lien frangible, ledit élément étant formé d'une seule pièce avec ladite gouttière.

En particulier, l'élément tubulaire forme un moyen de préhension, par exemple pour un bras robotisé dans le cadre d'une production en série industrielle, pour maintenir la gouttière 2 lors de sa solidarisation au corps 1. Ainsi, la présence d'un tel élément de préhension permet d'éviter la préhension de la gouttière 2 par sa pointe acérée 2a, et ainsi d'abimer le tranchant de ladite pointe. Un tel élément permet également d'éviter la préhension de la gouttière 2 par son extrémité proximale 2b, une telle préhension rendant difficile voire impossible la solidarisation de ladite gouttière sur le corps 1.

Une fois la gouttière 2 solidarisée au corps 1, l'élément tubulaire est séparé de ladite gouttière par rupture du lien frangible, par exemple en faisant pivoter ledit élément tubulaire par rapport à ladite gouttière autour dudit lien frangible.

Toutefois, après la rupture du lien frangible, des débris de matière peuvent rester sur l'extrémité proximale 2b de la gouttière 2, lesdits débris pouvant en outre être relativement tranchants et présenter un risque de blessure pour le praticien. Pour limiter le contact de tels débris avec les doigts du praticien, la paroi proximale 1b du corps central 1 peut présenter un renfoncement au-dessus duquel débouche l'extrémité proximale 2b de la gouttière 2, ledit renfoncement présentant une dimension suivant l'axe A de ladite gouttière qui est agencée pour éviter que de tels débris ne fassent saillie au-delà dudit renfoncement.

Les bras 4 de préhension peuvent également être formés en une seule pièce avec le corps central 1 et/ou la gouttière 2, notamment par des procédés tels que décrits précédemment.

Les bras 4 de préhension peuvent également être rapportés sur le corps central 1, notamment par surmoulage. En particulier, le corps central 1 peut être formé par emboutissage avec deux armatures latérales qui s'étendent symétriquement de part et d'autre du plan sagittal S, comme représenté sur les figures 8 à 11, puis lesdites armatures peuvent être revêtues d'un volume de matière, par exemple par surinjection de ladite matière sur lesdites armatures, afin de former les bras 4 de préhension.

Le dispositif ainsi fabriqué peut ensuite être conditionné avec au moins une canule 20 à bout arrondi 20a pour former un kit d'injection. Pour ce faire, le dispositif et la canule 20 d'un même kit peuvent être conditionnés dans un même sachet stérile transparent, notamment à base de polypropylène et/ou de polyéthylène, ledit sachet pouvant en outre être souple ou rigide et/ou comprendre au moins une portion à base de papier pelable.

Par ailleurs, le kit ainsi conditionné peut être stocké dans une boîte contenant d'autres kits également conditionnés en sachets, afin d'être protégés de l'humidité et de la lumière durant leur stockage.

En particulier, le corps central 1 du dispositif peut présenter une coloration identique à celle de l'embase 22 de la canule 20, ce qui permet d'identifier ledit dispositif et ladite canule comme appartenant à un même kit d'injection. De ce fait, le praticien peut identifier, parmi plusieurs kits conditionnés en sachets, le kit dont il a besoin juste en regardant la couleur de l'embase 22 et du corps central 1.

Le dispositif peut également être conditionné seul, notamment dans un sachet tel que décrit ci-dessus, afin d'être commercialisé seul, notamment pour la vente à des laboratoires.

Par ailleurs, après le conditionnement en sachet, le dispositif ou le kit dispositif/canule 20 sont stérilisés, par exemple au moyen d'un procédé de stérilisation par oxyde d'éthylène (ETO), par rayonnement ionisant ou par vapeur.

De plus, le sachet peut comprendre des indications pour le praticien, comme un numéro de lot, les dimensions de la canule 20 et/ou du dispositif, une notion restreignant l'usage à des praticiens qualifiés, une référence à l'organisme notifié et/ou un marquage de conformité à une norme, notamment la norme CE.

En particulier, le sachet peut comprendre des informations utiles pour une utilisation sans danger du dispositif ou du kit dispositif/canule 20 qu'il contient, par exemple une date de stérilisation et/ou une date de péremption. Par ailleurs, si le sachet présente un mauvais état et/ou une ouverture, le praticien peut savoir immédiatement que le dispositif ou le kit contenu dans ledit sachet doit être détruit pour la sécurité du patient.

## Revendications

1. Dispositif pour l'introduction d'une canule (20) à bout arrondi (20a) sous la peau d'un patient en vue de l'injection d'un produit biocompatible, ledit dispositif comprenant un corps central (1) équipé d'une gouttière (2) qui s'étend suivant un plan sagittal (S) en présentant une pointe distale acérée (2a) faisant saillie depuis une extrémité distale (1a) dudit corps pour le perçage de ladite peau, ladite gouttière étant agencée pour permettre le coulissement sur elle de la canule (20) au-delà de ladite pointe distale vers au moins une zone à traiter située sous ladite peau, ledit dispositif étant **caractérisé en ce que** le corps central (1) est pourvu de deux bras latéraux (4) de préhension qui s'étendent symétriquement de part et d'autre du plan sagittal (S) suivant une direction transversale (D) qui forme avec ledit plan sagittal un angle (α) compris entre 30° et 85°, chaque bras (4) de préhension présentant en outre une surface de tenue digitale (5) qui présente une portion centrale avant (5a) située à une distance (d) comprise entre 8 mm et 30 mm de l'extrémité distale (1a) du corps central (1), le corps central (1) présentant une géométrie en forme de V, chacun des deux bras latéraux (4) de préhension comprenant une extrémité proximale (4a) qui présente une pente (9), le corps central (1) comprenant en outre une paroi supérieure (1c) qui présente une zone de dégagement (10) et forme un sillon évasé au centre duquel la gouttière (2) est disposée, la zone de dégagement (10) étant agencée pour faciliter la disposition de la canule (20) sur la gouttière (2), la zone de dégagement (10) étant formée par le sillon évasé de la paroi supérieure (1c) et par les pentes (9) des extrémités proximales (4) de chacun des bras de préhension (4), la gouttière (2) présentant une fente supérieure (3) qui est entourée par la paroi supérieure (1c) du corps central (1) et qui s'étend sur toute la longueur de la gouttière (2), ladite gouttière s'étendant depuis sa pointe distale (2a) qui fait saillie depuis une extrémité distale (1a) du corps central (1) jusqu'à une paroi proximale (1b) du corps central (1).

2. Dispositif selon la revendication 1, **caractérisé en ce que** la gouttière (2) définit un plan transversal (T) qui est perpendiculaire au plan sagittal (S) et sur lequel ladite gouttière s'étend jusqu'à sa pointe distale (2a), ledit plan transversal passant par ladite pointe distale, le corps central (1) présentant une partie inférieure (1d) qui s'étend sous ledit plan transversal en formant avec ledit plan transversal un angle (β) inférieur ou égal à 15°.

3. Dispositif selon l'une des revendications 1 ou 2, **caractérisé en ce que** la gouttière (2) présente une extrémité proximale ouverte (2b) qui débouche dans une paroi proximale (1b) du corps central (1), ladite paroi proximale étant entourée d'une zone de dégagement arrière (11).

4. Dispositif selon l'une quelconque des revendications 1 à 3, **caractérisé en ce que** l'extrémité distale (1a) du corps central (1) présente une forme émoussée, et notamment arrondie.

5. Dispositif selon l'une quelconque des revendications 1 à 4, **caractérisé en ce que** chaque bras (4) de préhension présente une géométrie essentiellement cylindrique autour de sa direction transversale (D).

6. Dispositif selon l'une quelconque des revendications 1 à 5, **caractérisé en ce que** chaque surface (5) de tenue digitale présente un méplat avant (7) sur lequel est formée la portion centrale avant (5a).

7. Dispositif selon l'une quelconque des revendications 1 à 6, **caractérisé en ce que** la gouttière (2) définit un plan frontal qui s'étend perpendiculairement au plan sagittal (S), les bras (4) de préhension s'étendant l'un par rapport à l'autre dans ledit plan frontal suivant un angle (γ) compris entre 90° et 180°.

8. Kit d'injection d'un produit biocompatible, ledit kit comprenant un dispositif d'introduction selon l'une quelconque des revendications 1 à 7 ainsi qu'au moins une canule d'injection (20) à bout arrondi (20a), la gouttière (2) étant agencée pour permettre le coulissement sur elle de ladite au moins une canule au-delà de sa pointe distale (2a) vers au moins une zone à traiter située sous la peau d'un patient.

9. Kit d'injection selon la revendication 8, **caractérisé en ce que** la canule d'injection (20) présente au moins un orifice d'injection (21), ledit orifice étant disposé latéralement à proximité du bout arrondi (20a).

10. Kit d'injection selon l'une des revendications 8 ou 9, **caractérisé en ce que** la gouttière (2) présente une dimension interne qui est supérieure au calibre externe de la canule d'injection (20) devant coulisser sur elle.

## Patentansprüche

1. Vorrichtung zum Einführen einer Kanüle (20) mit abgerundeter Kuppe (20a) unter die Haut eines Patienten zwecks Injektion eines biokompatiblen Produktes, wobei die Vorrichtung einen zentralen Körper (1) umfasst, der mit einer Rinne (2) ausgerüstet ist, die sich entlang einer Sagittalebene (S) erstreckt, und dabei eine scharfkantige distale Spitze (2a) aufweist, die aus einem distalen Ende (1a) des Körpers zum Durchdringen der Haut hervorsteht, wobei die Rinne angeordnet ist, um darauf das Gleiten der Kanüle (20) über die distale Spitze hinaus zu mindestens einem zu behandelnden Bereich zu ermöglichen, der sich unter der Haut befindet, wobei die Vorrichtung **dadurch gekennzeichnet ist, dass** der zentrale Körper (1) mit zwei seitlichen Greifarmen (4) versehen ist, die sich symmetrisch beiderseits der Sagittalebene (S) entlang einer Querrichtung (D) erstrecken, die mit der Sagittalebene einen Winkel (*α*) bildet, der zwischen 30° und 85° liegt, wobei jeder Greifarm (4) weiter eine Fingerhaltefläche (5) aufweist, die einen vorderen zentralen Abschnitt (5a) aufweist, der sich in einem Abstand (d) befindet, der zwischen 8 mm und 30 mm von dem distalen Ende (1a) des zentralen Körpers (1) liegt, wobei der zentrale Körper (1) eine Geometrie in Form eines V aufweist, jeder der beiden seitlichen Greifarme (4) ein proximales Ende (4a) aufweist, das eine Neigung (9) aufweist, der zentrale Körper (1) weiter eine obere Wand (1c) umfasst, die einen Freiraumbereich (10) aufweist, und eine ausgestellte Furche bildet, in deren Zentrum die Rinne (2) angeordnet ist, wobei der Freiraumbereich (10) angeordnet ist, um die Anordnung der Kanüle (20) auf der Rinne (2) zu erleichtern, wobei der Freiraumbereich (10) durch die ausgestellte Furche der oberen Wand (1c) und durch die Neigungen (9) der proximalen Enden (4) eines jeden der Greifarme (4) gebildet wird, wobei die Rinne (2) einen oberen Spalt (3) aufweist, der von der oberen Wand (1c) des zentralen Körpers (1) umgeben ist, und sich über die gesamte Länge der Rinne (2) erstreckt, wobei sich die Rinne aus ihrer distalen Spitze (2a), die aus einem distalen Ende (1a) des zentralen Körpers (1) hervorsteht, bis zu einer proximalen Wand (1b) des zentralen Körpers (1) erstreckt.

2. Vorrichtung nach Anspruch 1, **dadurch gekennzeichnet, dass** die Rinne (2) eine Querebene (T) definiert, die senkrecht zur Sagittalebene (S) liegt, und auf der sich die Rinne bis zu ihrer distalen Spitze (2a) erstreckt, wobei die Querebene über die distale Spitze verläuft, wobei der zentrale Körper (1) einen unteren Teil (1d) aufweist, der sich unter der Querebene erstreckt, und dabei mit der Querebene einen Winkel (β) kleiner oder gleich 15° bildet.

3. Vorrichtung nach einem der Ansprüche 1 oder 2, **dadurch gekennzeichnet, dass** die Rinne (2) ein offenes proximales Ende (2b) aufweist, das in eine proximale Wand (1b) des zentralen Körpers (1) mündet, wobei die proximale Wand von einem hinteren Freiraumbereich (11) umgeben ist.

4. Vorrichtung nach einem der Ansprüche 1 bis 3, **dadurch gekennzeichnet, dass** das distale Ende (1a) des zentralen Körpers (1) eine stumpfe, und insbesondere abgerundete Form aufweist.

5. Vorrichtung nach einem der Ansprüche 1 bis 4, **dadurch gekennzeichnet, dass** jeder Greifarm (4) eine im Wesentlichen zylindrische Geometrie um seine Querrichtung (D) aufweist.

6. Vorrichtung nach einem der Ansprüche 1 bis 5, **dadurch gekennzeichnet, dass** jede Fingerhaltefläche (5) eine vordere Abflachung (7) aufweist, auf der der vordere zentrale Abschnitt (5a) gebildet ist.

7. Vorrichtung nach einem der Ansprüche 1 bis 6, **dadurch gekennzeichnet, dass** die Rinne (2) eine vordere Ebene definiert, die sich senkrecht zur Sagittalebene (S) erstreckt, wobei sich die Greifarme (4) in der vorderen Ebene in Bezug zueinander entsprechend einem Winkel (γ) erstrecken, der zwischen 90° und 180° liegt.

8. Injektionsset für ein biokompatibles Produkt, wobei das Set eine Vorrichtung zum Einführen nach einem der Ansprüche 1 bis 7, sowie mindestens eine Injektionskanüle (20) mit abgerundeter Kuppe (20a) umfasst, wobei die Rinne (2) angeordnet ist, um darauf das Gleiten der mindestens einen Kanüle über ihre distale Spitze (2a) hinaus zu mindestens einem zu behandelnden Bereich zu ermöglichen, der sich unter der Haut eines Patienten befindet.

9. Injektionsset nach Anspruch 8, **dadurch gekennzeichnet, dass** die Injektionskanüle (20) mindestens eine Injektionsöffnung (21) aufweist, wobei die Öffnung seitlich in der Nähe der abgerundeten Kuppe (20a) angeordnet ist.

10. Injektionsset nach einem der Ansprüche 8 oder 9, **dadurch gekennzeichnet, dass** die Rinne (2) eine Innenabmessung aufweist, die größer als das äußere Kaliber der Injektionskanüle (20) ist, die darauf gleiten soll.

## Claims

1. Device for inserting a blunt-tip (20a) cannula (20) under the skin of a patient with a view to injecting a biocompatible product, said device comprising a central body (1) equipped with a groove (2) that extends along a sagittal plane (S) while having a sharp distal tip (2a) projecting from a distal end (1a) of said body in order to pierce said skin, said groove being arranged to allow the cannula (20) to slide along same beyond said distal tip towards at least one area to be treated located under said skin, said device being **characterised in that** the central body (1) is provided with two lateral grasping arms (4) that extend symmetrically on either side of the sagittal plane (S) in a transverse direction (D) that forms, with said sagittal plane, an angle (α) of between 30° and 85°, each gripping arm (4) further having a finger grip surface (5) that has a front central portion (5a) located at a distance (d) between 8 mm and 30 mm from the distal end (1a) of the central body (1), the central body having a V-shaped geometry, each of the two lateral grasping arms (4) comprising a proximal end (4a) which has a slope (9), the central body comprising furthermore an upper wall (1c) which has a clearance zone (10) and forms a flared channel at the center of which the groove (2) is arranged, the clearance zone (10) being arranged to facilitate the placement of the cannula (20) on the groove (2), the clearance zone (10) being formed by the flared channel of the upper wall (1c) and by the slopes (9) of the proximal ends (4) of each of the grasping arms (4), the groove (2) having an upper slot (3) which is surrounded by the upper wall (1c) of the central body (1) and that extends over the entire length of the groove (2), said groove extending from its distal tip (2a) which protrudes from a distal end (1a) of the central body (1) to a proximal wall (1b) of the central body (1).

2. Device according to claim 1, **characterised in that** the groove (2) defines a transverse plane (T) which is perpendicular to the sagittal plane (S) and whereon said groove extends to its distal tip (2a), said transverse plane passing through said distal tip, with the central body (1) having a lower portion (1d) that extends under said transverse plane by forming with said transverse plane an angle (β) less than or equal to 15°.

3. Device according to one of claims 1 or 2, **characterised in that** the groove (2) has an open proximal end (2b) that opens into a proximal wall (1b) of the central body (1), said proximal wall being surrounded by a rear clearance zone (11).

4. Device according to any of claims 1 to 3, **characterised in that** the distal end (1a) of the central body (1) has a blunt shape, and in particular rounded.

5. Device according to any of claims 1 to 4, **characterised in that** each grasping arm (4) has a substantially cylindrical geometry around its transverse direction (D).

6. Device according to any of claims 1 to 5, **characterised in that** each finger grip surface (5) has a front flat surface (7) whereon is formed the front central portion (5a).

7. Device according to any of claims 1 to 6, **characterised in that** the groove (2) defines a frontal plane that extends perpendicularly to the sagittal plane (S), with the grasping arm (4) extending in relation to one another in said frontal plane according to an angle (γ) between 90° and 180°.

8. Kit for injecting a biocompatible product, said kit comprising a device for inserting according to any of claims 1 to 7 as well as at least one blunt-tip (20a) injection cannula (20), the groove (2) being arranged to allow for the sliding on same of said at least one cannula beyond its distal tip (2a) towards at least one area to be treated located under the skin of a patient.

9. Kit for injecting according to claim 8, **characterised in that** the injection cannula (20) has at least one injection orifice (21), said orifice being arranged laterally in the vicinity of the blunt tip (20a).

10. Kit for injecting according to one of claims 8 or 9, **characterised in that** the groove (2) has an internal dimension that is greater than the external calibre of the injection cannula (20) that has to slide over it.
